# FASCICULE DE BREVET EUROPEEN

(11) **EP 0 737 100 B1**
(45) Date de publication et mention de la délivrance du brevet: **23.09.1998**
(21) Numéro de dépôt: 95903845.6
(22) Date de dépôt: 16.12.1994
(51) Int. Cl.: B01J 25/02, B01J 25/00

(54) **CATALYSEUR D'HYDROGENATION DE NITRILES EN AMINES, SON PROCEDE DE PREPARATION ET PROCEDE D'HYDROGENATION EN FAISANT APPLICATION**
KATALYSATOR ZUR HYDRIERUNG VON NITRILEN IN AMINEN, VERFAHREN ZUR HERSTELLUNG DES KATALYSATORS UND VERFAHREN ZUR HYDRIERUNG UNTER VERWENDUNG DESSELBEN
CATALYST FOR HYDROGENATING NITRILES INTO AMINES, METHOD FOR ITS PREPARATION AND HYDROGENATION METHOD USING SAID CATALYST

(30) Priorité: 28.12.1993 FR 9316006
(43) Date de publication de la demande: 16.10.1996
(73) Titulaire: RHODIA FIBER & RESIN INTERMEDIATES, 92408 Courbevoie Cédex (FR)
(72) Inventeur: CORDIER, Georges, F-69340 Francheville (FR); FOUILLOUX, Pierre, F-69300 Caluire-et-Cuire (FR); LAURAIN, Nathalie, F-69003 Lyon (FR)
(74) Mandataire: Vignally, Noel
(86) Numéro de dépôt international: FR9401476
(87) Numéro de publication internationale: WO9517959

(56) Documents cités:
- EP-A- 0 223 035
- GB-A- 2 104 794
- US-A- 4 429 159
- Scientific Papers I.P.C.R., Tokyo, vol. 55, pp. 105-108 (1961)
- Applied Catalysis, vol. 49, pp. 91-99 (1989)

## Description

Le domaine de l'invention est celui de la réduction catalytique de nitriles en amines.

Plus précisément, il est ici question de l'hydrogénation catalytique de nitriles à l'aide des catalyseurs du type Nickel de RANEY, c'est-à-dire obtenus par attaque alcaline d'un alliage précurseur Ni/Al.

La présente invention concerne particulièrement un catalyseur d'hydrogénation de nitriles, notamment de dinitriles en diamines, ledit catalyseur étant de type Nickel de RANEY dopé par au moins un élément choisi parmi les groupes IIB, IVB à VIIB de la classification périodique.

La présente invention a également pour objet un procédé de préparation d'un catalyseur d'hydrogénation de nitriles en amines de type Ni de RANEY dopé, tel que celui visé ci-dessus.

La présente invention est, en outre, relative à un procédé d'hydrogénation de nitriles en amines faisant application des catalyseurs ci-dessus considérés.

Au sens de l'invention, on désigne par nitriles tous les mono et dinitriles aromatiques et/ou aliphatiques et, en particulier mais non limitativement, les dinitriles résultant d'acides dicarboxyliques, de préférence de C3 à C6 tels que l'adiponitrile, le glutaronitrile, les succinonitrile, le malononitrile, éventuellement substitués par des groupements alkyles inférieurs ayant de 1 à 6 atomes de carbone, tels que notamment méthyle ou éthyle.

Les nickels de RANEY sont des catalyseurs largement utilisés dans l'industrie et les laboratoires pour les réactions d'hydrogénation. Ils sont préparés par attaque alcaline d'alliages Al/Ni riches en aluminium. Le catalyseur est constitué par des agglomérats de cristallites de nickel à grande surface spécifique et à contenu en aluminium résiduel variable.

Dans le domaine de la catalyse en général et en catalyse d'hydrogénation en particulier, les objectifs sans cesse visés sont, d'une part, l'optimisation de l'activité et de la sélectivité du catalyseur vis-à-vis du substrat à hydrogéner et, d'autre part, la stabilité.

Il va de soi que ces trois paramètres clés sont indissociables des autres contraintes que sont la facilité de fabrication du catalyseur, son faible coût de revient, ainsi que la souplesse et la commodité de sa mise en oeuvre en hydrogénation.

Il a ainsi déjà été proposé de doper les catalyseurs nickel de RANEY à l'aide de promoteurs tels que le titane, le chrome, le fer, le cobalt, le cuivre, le molybdène, le tantale, le zirconium ou d'autres métaux, en vue d'améliorer leur activité et/ou leur sélectivité.

Ces promoteurs ont pour fonction de modifier les facteurs structuraux électroniques du nickel de RANEY. Classiquement, ils sont ajoutés à l'alliage Ni/Al en fusion, conformément à une technique de dopage dite "métallurgique".

Le brevet FR 913 997 décrit notamment l'utilisation d'un Ni de RANEY, préparé à partir d'un alliage Al/Ni contenant de 0,5 % à 3,5 % en poids de chrome par rapport au nickel, pour l'hydrogénation de l'adiponitrile en hexaméthylènediamine.

Le document B.N. TYUTYUNNIKOV et al- The Soviet Chemical Industry, N^{o} 6, Juin 1991, ainsi que l'article L.Kh FREIDLIN et al - Russian Chemical Reviews - vol 33 N^{o} 6 - Juin 1964, parmi d'autres, concernent la réduction catalytique de nitriles aliphatiques (dinitriles) à l'aide de catalyseurs d'hydrogénation de RANEY, promus à l'aide de dopants métalliques.

De tels catalyseurs sont relativement difficiles à obtenir, car ils nécessitent la mise en oeuvre de moyens d'amenée et de maintien en fusion de l'alliage précurseur. En outre, les procédés d'obtention de ces catalyseurs connus sont relativement peu souples, notamment au regard du réglage de la quantité de dopants introduits dans l'alliage. En effet, le mélange de l'alliage précurseur avec le dopant peut, parfois, ne pas être homogène et générer ainsi des variations importantes de composition ou de teneur en dopant dans la masse du catalyseur. Il faut également signaler que l'on ne maîtrise pas les phénomènes qui interviennent dans le fondu. On peut ainsi assister, dans certains cas, à la formation de structures complexes cristallographiques, nuisibles aux performances catalytiques du produit.

De plus, il existe certaines limitations quant au choix de la nature du dopant en raison de problèmes de compatibilité avec le nickel et l'aluminium en fusion.

Il convient aussi de souligner que certains des catalyseurs Ni de RANEY dopés par voie métallurgique ne permettent pas toujours d'obtenir des résultats satisfaisants quant à l'activité, la sélectivité et la stabilité dans le temps.

Il est également à noter que les catalyseurs connus décrits par TYUTYUNNIKOV et al ainsi que par FREIDLIN présentent des teneurs en aluminium résiduel relativement importantes, c'est-à-dire supérieures à 6 % en poids. Cela n'est pas pour favoriser les performances du catalyseur. Enfin, on observe qu'ils se caractérisent par un rapport pondéral dopant/Ni supérieur à 8 %.

L'article de TAKAGI et al publié dans Scientific Papers I.P.C.R., TOKYO, 55, pages 105 - 108 (1961) décrit un procédé de préparation de catalyseurs d'hydrogénation de différents composés comportant des groupes carbonyle, nitro, nitrile ou des doubles liaisons éthyléniques. Ce procédé consiste à ajouter une solution aqueuse d'un sel métallique, tel que nitrate de cuivre, nitrate de chrome, molybdate d'ammonium, tungstate de sodium, chlorure ferrique ou nitrate de cobalt, à la solution de soude lors de l'attaque de l'alliage nickel-aluminium. Lors de l'hydrogénation de l'acétonitrile, l'addition de ces sels, hormis le sel ferrique, augmente l'activité du catalyseur.

La présente invention vise à pallier les lacunes et les inconvénients des catalyseurs d'hydrogénation du type RANEY dopés de l'art antérieur.

Elle a donc pour objectif de fournir un catalyseur Ni de RANEY d'hydrogénation dopé, qui soit de préparation facile et peu onéreuse et qui satisfasse aux exigences d'activité, de sélectivité et de stabilité dans le temps.

Un autre objectif de l'invention est de fournir un procédé de préparation d'un catalyseur Ni de RANEY d'hydrogénation, qui soit de mise en oeuvre simple et économique.

Un autre objectif de l'invention est de fournir un procédé d'hydrogénation dans lequel on a recours à un catalyseur Ni de RANEY du type de celui visé ci-dessus.

La demanderesse a eu le mérite de mettre en évidence qu'il convenait d'effectuer un dopage chimique de l'alliage précurseur au cours de l'attaque alcaline, pour atteindre ces objectifs.

Il s'ensuit que la présente invention a pour objet un catalyseur d'hydrogénation de nitriles en amines, de type Ni de RANEY et dopé par au moins un élément choisi parmi les éléments des groupes IIB, IVB à VIIB de la classification périodique, caractérisé en ce que son alliage précurseur est sensiblement exempt de dopant avant l'attaque alcaline.

Au sens de l'invention, le terme "dopant" correspond à un élément chimique additionné volontairement à l'alliage précurseur brut, immédiatement après sa synthèse métallurgique, en vue d'améliorer les propriétés catalytiques.

Un tel dopage chimique amène une grande simplification dans l'obtention du catalyseur, ainsi qu'une amélioration notable de la qualité de la catalyse.

En particulier, ce nouveau catalyseur permet d'augmenter la productivité et la sélectivité de l'hydrogénation, tout en permettant une diminution du taux d'apparition des impuretés ou sous-produits d'hydrogénation indésirables.

Selon un autre aspect de l'invention, ce catalyseur comprend une teneur en aluminium, exprimée en poids par rapport au poids du nickel, inférieure ou égale à 6 %, de préférence inférieure ou égale à 5 % et, plus préférentiellement encore, comprise entre 2,5 % et 4,5 %.

Avantageusement, le rapport pondéral dopant/Ni de ce catalyseur est compris entre 0,05 % et 10 %, de préférence entre 0,1 % et 5 % et, plus préférentiellement encore, entre 0,3 % et 3,5 %.

S'agissant du dopant, il est, de préférence, choisi parmi les éléments suivants : titane, chrome, zirconium, vanadium, molybdène, manganèse, zinc. Le titane, le chrome et le zirconium sont particulièrement préférés.

Outre le nickel, l'aluminium et le (ou les) dopant(s), le catalyseur peut contenir des éléments métalliques de constitution comme, par exemple, le fer. Ce ou ces éléments métalliques de constitution sont présents dans une quantité inférieure ou égale à 10 %, de préférence inférieure ou égale à 7 % et, plus préférentiellement encore, inférieure ou égale à 5,5 % en poids dans le catalyseur fini.

La présente invention concerne également un procédé de préparation d'un catalyseur du type Ni de RANEY utilisable pour l'hydrogénation des nitriles en amines, ledit catalyseur étant dopé avec au moins un élément choisi parmi les éléments des groupes IIB, IVB à VIIB de la classification périodique.

De façon conventionnelle, la préparation d'un catalyseur du type Nickel de RANEY consiste à soumettre un alliage métallique comprenant du nickel et de l'aluminium à une attaque alcaline, conduisant à la lixiviation de la plus grande partie de l'aluminium.

Conformément à l'invention, l'alliage précurseur métallique Ni/Al de départ n'a pas subi de dopage par la voie métallurgique. Il est donc sensiblement vierge d'élément métallique promoteur.

On effectue alors l'attaque alcaline de l'alliage précurseur en présence du dopant sous forme complexée, qui est destiné à se lier au nickel par voie chimique.

Le dopage chimique selon l'invention, ne complique absolument pas la préparation traditionnelle d'un catalyseur de RANEY.

En outre et de manière tout à fait surprenante et inattendue, il apparaît que cette disposition simple de procédé permet d'aboutir à une structure catalytique de faible coût de revient et très performante.

Conformément à une caractéristique préférée de l'invention, le dopant est introduit dans le milieu d'attaque alcaline, sous forme d'une solution, de préférence alcaline et, plus préférentiellement encore, de même nature et sensiblement de même titre alcalin que le milieu d'attaque.

Avantageusement, le vecteur utilisé pour transporter le dopant et pour l'amener en contact avec l'alliage précurseur est un complexe du dopant avec au moins un chélatant. Il peut s'agir par exemple d'un sel du dopant, de préférence soluble dans le milieu d'attaque alcaline. Le chélatant mis en oeuvre est, de préférence, sélectionné au sein des dérivés carboxyliques, des triènes, des amines ou autres séquestrants appropriés.

En ce qui concerne les dérivés d'acides carboxyliques, on retiendra plus volontiers les composés suivants : tartrate, citrate, éthylènediaminetétraacétate, gluconate, carboxylates d'acides gras tels que stéarate par exemple.

Conformément à un mode de mise en oeuvre particulièrement avantageux, on sélectionne le tartrate en tant qu'agent chélatant du ou des dopants chimiques de l'alliage Ni-Al.

Les éléments dopants considérés sont les mêmes que ceux définis précédemment.

Concernant la chronologie du procédé, le dopant est de préférence introduit dans le réacteur dès le début de la lixiviation et en pratique en même temps que l'alliage à traiter.

Ainsi, dans le cas où le milieu d'attaque alcaline est constitué, par exemple, par de la soude 6N, le dopant sous forme chélatée et lui aussi solubilisé dans de la soude 6N, est amené dans le milieu réactionnel simultanément à l'alliage.

Selon une variante de mise en oeuvre du procédé selon l'invention, le dopage chimique est effectué à l'aide de deux éléments métalliques d'admission choisis dans les groupes IIB, IVB à VIIB de la classification périodique, de préférence parmi les éléments suivants : titane, chrome, zirconium, vanadium, molybdène, manganèse, zinc. L'association de titane et de chrome est particulièrement préférée.

La quantité de dopant mise en oeuvre lors de l'attaque alcaline, est fonction de la concentration finale en dopant visée dans le catalyseur. L'homme du métier est tout à fait à même d'ajuster le titre de la solution alcaline de dopant, en tenant compte des volumes mis en oeuvre, de la concentration finale visée en dopant ainsi que de la limite de solubilité du complexe dopant/chélatant dans le milieu alcalin considéré.

Il est en effet préférable que, dans les conditions réactionnelles, le complexe dopant/chélatant se trouve sous forme solubilisée, lors de son introduction dans le milieu réactionnel d'attaque alcaline.

Sachant que c'est la solubilité qui fixe la limite supérieure de concentration en dopant dans le milieu réactionnel, on peut indiquer, pour fixer les idées, que cette concentration est par exemple supérieure ou égale à 10⁻⁵ mole/litre et de préférence supérieure ou égale à 10⁻³ mole/litre de milieu réactionnel à 25°C.

L'introduction de dopant mise à part, le procédé selon l'invention s'assimile aux méthodologies classiques d'attaque alcaline d'alliage précurseur Ni-Al, pour obtenir un catalyseur Ni de RANEY d'hydrogénation.

Ce procédé est l'une des techniques envisageables pour préparer le nouveau catalyseur dopé conforme à l'invention et décrit ci-avant.

La présente invention concerne également un procédé d'hydrogénation de nitriles en amines, dans lequel on met en oeuvre le nouveau catalyseur selon l'invention.

Ce procédé s'applique, plus particulièrement mais non limitativement, aux substrats nitriles de formule (I) :

NC―R―CN (I)

dans laquelle R représente un groupement alkylène ou alcénylène, linéaire ou ramifié, ayant de 1 à 12 atomes de carbone, ou un groupement arylène ou aralkylène ou aralcénylène substitué ou non.

De préférence, on met en oeuvre dans le procédé de l'invention des dinitriles de formule (I) dans laquelle R représente un radical alkylène, linéaire ou ramifié ayant de de 2 à 6 atomes de carbone.

A titre d'exemples de tels dinitriles, on peut citer notamment l'adiponitrile, le méthylglutaronitrile, l'éthylsuccinonitrile, le malononitrile, le succinonitrile et le glutaronitrile et leurs mélanges, notamment les mélanges adiponitrile, méthylglutaronitrile, éthylsuccinonitrile qui proviennent d'un même procédé de synthèse de l'adiponitrile.

L'introduction du substrat nitrile, par exemple l'adiponitrile, dans le milieu réactionnel se fait en respectant une concentration comprise entre 0,001 % et 30 % en poids par rapport au poids total (p/p) du milieu réactionnel et de préférence entre 0,1 % et 20 % p/p.

De façon privilégiée, la base forte mise en oeuvre est choisie parmi les composés suivants : LiOH, NaOH, KOH, RbOH, CsOH et leur mélanges.

En pratique, on utilise préférentiellement NaOH et KOH, pour un bon compromis performance-prix, bien que RbOH et CsOH donnent des résultats encore meilleurs.

Le milieu réactionnel d'hydrogénation est de préférence liquide. Il contient au moins un solvant apte à solubiliser le substrat nitrile à hydrogéner, sachant que cette transformation s'opère mieux lorsque ledit substrat se trouve en solution.

Suivant une modalité intéressante du procédé selon l'invention, on utilise un milieu réactionnel liquide au moins partiellement aqueux. L'eau est généralement présente dans une quantité inférieure ou égale à 50 %, avantageusement inférieure ou égale à 20 % en poids par rapport au milieu réactionnel total. Plus préférentiellement encore, la teneur en eau du milieu réactionnel est comprise entre 0,1 et 15 % en poids par rapport à l'ensemble des constituants dudit milieu.

En complément ou en substitution à l'eau, on peut prévoir au moins un autre solvant, du type alcool et/ou amide. Les alcools qui conviennent plus particulièrement sont par exemple le méthanol, l'éthanol, le propanol, l'isopropanol, le butanol, les glycols, tels que l'éthylène et/ou le propylène glycol, des polyols et/ou des mélanges desdits composés.

Dans le cas où le solvant est constitué par un amide, il peut s'agir par exemple du diméthylformamide ou du diméthylacétamide.

Lorsqu'il est employé avec l'eau, le solvant, de préférence alcoolique, représente de deux à quatre parties en poids pour une partie en poids d'eau et de préférence trois parties pour une partie d'eau.

Selon une autre caractéristique préférée de l'invention, on incorpore de l'amine, dont la préparation est visée par le procédé, au sein du milieu réactionnel. Il s'agit par exemple d'hexaméthylènediamine, lorsque le substrat nitrile est l'adiponitrile.

La concentration de l'amine visée dans le milieu réactionnel est avantageusement comprise entre 50 % et 99 % en poids par rapport à la totalité du solvant inclus dans ledit milieu réactionnel et, plus préférentiellement encore, est comprise entre 60 % et 99 % en poids.

La quantité de base dans le milieu réactionnel varie en fonction de la nature du milieu réactionnel.

Dès lors que le milieu réactionnel ne contient que de l'eau et de l'amine visée, à titre de milieu solvant liquide, la quantité de base est avantageusement supérieure ou égale à 0,1 mol/kg de catalyseur, de préférence comprise entre 0,1 et 2 mol/kg de catalyseur et plus préférentiellement encore entre 0,5 et 1,5 mol/kg de catalyseur.

Dans le cas où le milieu réactionnel comprend de l'eau et un alcool et/ou un amide, la quantité de base est supérieure ou égale à 0,05 mol/kg de catalyseur, est comprise de préférence entre 0,1 et 10,0 mol/kg et plus préférentiellement encore entre 1,0 et 8,0 mol/kg.

Une fois arrêtée la composition du milieu réactionnel et le choix du catalyseur, on procéde à un mélange de ces deux éléments, puis on chauffe ce mélange à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 0,10 et 10 MPa.

La durée de la réaction est variable en fonction des conditions réactionnelles et du catalyseur.

Dans un mode de fonctionnement discontinu, elle peut varier de quelques minutes à plusieurs heures.

Dans un mode de fonctionnement continu, qui est parfaitement envisageable pour le procédé selon l'invention, la durée n'est évidemment pas un paramètre figeable.

Il est à noter que l'homme du métier peut moduler la chronologie des étapes du procédé selon l'invention, selon les conditions opératoires. L'ordre donné ci-avant ne correspond qu'à une forme préférée, mais non limitative, du procédé selon l'invention.

Les autres conditions qui régissent l'hydrogénation (en mode continu ou discontinu) conforme à l'invention, relèvent de dispositions techniques traditionnelles et connues en elles-mêmes.

Grâce à toutes les dispositions avantageuses évoquées ci-dessus, le procédé de l'invention permet d'hydrogéner des substrats nitriles en amines, de façon sélective, rapide, commode et économique.

L'hydrogénation d'adiponitrile en hexaméthylènediamine est particulièrement importante pour les producteurs de polyamide-6,6, puisque ce dérivé hydrogéné est l'un des monomères de base de cette synthèse industrielle de grande envergure.

L'hydrogénation des dinitriles peut également donner accès à des aminonitriles. Ainsi, il est possible d'hydrogéner une seule des deux fonctions nitriles de l'adiponitrile pour obtenir l'aminocapronitrile. Ce dernier composé est aisément transformable par hydrolyse cyclisante en caprolactame, qui est le produit de départ d'une autre grande synthèse industrielle de polyamide, à savoir le polyamide-6.

Il s'ensuit que ce nouveau catalyseur d'hydrogénation simple à préparer, économique, plus sélectif, plus actif et plus stable que les catalyseurs connus, représente un progrès technique notable et appréciable dans ce domaine.

L'invention sera mieux comprise, ses avantages ainsi que ses variantes de mise en oeuvre ressortiront bien des exemples qui suivent, de préparation du nouveau catalyseur considéré et d'application de celui-ci dans l'hydrogénation d'adiponitrile en hexaméthylènediamine.

### EXEMPLES

Les alliages précurseurs de départ sont issus de quatre phases typiques du mélange binaire Nickel - Aluminium : NiAl₃, Ni₂Al₃, l'eutectique Al/NiAl₃ et un proeutectique Al/NiAl₃.

Les alliages utilisés dans les exemples sont les suivants :
- l'alliage commercial Ni/Al = 50/50 en poids (mélange NiAl₃ + Ni₂Al₃, eutectique Al/NiAl₃) ;
- une phase pure NiAl₃ homogénéisée dans laquelle Ni/Al = 42/58 en poids ;
- un brut de coulée proeutectique dans lequel Ni/Al = 28/72 en poids ;
- et un brut de coulée eutectique dans lequel Ni/Al = 6/94 en poids.

### MODE DE PREPARATION D'UN CATALYSEUR Ni DE RANEY DOPE CHIMIQUEMENT

### 1 OBTENTION DES SOLUTIONS DE DOPAGE

### 1.1. Préparation d'une solution de tartrate de titane +IV

Dans un bécher de 500 ml, on pèse 120 g d'acide L.tartrique. On ajoute 340 g d'eau distillée. On agite jusqu'à totale dissolution du solide.

La préparation de tartrate de titane s'effectue dans une boîte à gants purgée plusieurs fois à l'argon.

Pour ce faire, on verse à l'aide d'une pipette une quantité de 12,59 g de TiCl₄ dans la solution d'acide tartrique. On observe toujours un important dégagement d'HCl avec un précipité de TiO₂. On pèse la quantité de TiCl₄ introduit. La solution est très trouble (couleur blanche). On transvase dans une fiole de 1000 ml et on complète le volume à 1000 ml par ajout d'une solution de NaOH 6N. La solution est toujours très trouble. On attend quelques heures que l'équilibre s'établisse. Après 6 heures, on dispose d'une solution limpide à 3,16 g/l en Ti+IV.

### 1.2. Préparation de tartrate de chrome +III

Dans un erlen de 1000 ml, on pèse 140,16 g d'acide L.tartrique. On ajoute 267,72g d'eau distillée. On agite pour dissoudre le solide. On introduit ensuite, à la spatule, 15,09 g d'acétate hydroxyde de chrome +III (CH₃CO₂)₇ Cr₃ (OH)₂. On agite jusqu'à ce que le solide soit complètement dissous. On ajoute ensuite de la soude 6 N jusqu'à un volume de 1000 ml.

La solution obtenue titre 3,90 g/l en Cr⁺ III.

### 2. PREPARATION DU CATALYSEUR Ni RANEY

Pour les quatre types d'alliage précurseur Ni-Al mis en oeuvre indiqués précédemment, la méthodologie suivante est utilisée :
- **Etape 1** :: Dans un ballon en téflon de 2 l on introduit 300 ml de soude 6N à température ambiante.
- **Etape 2 :**: On pèse 10,00 g d'alliage dans un bécher.
- **Etape 3 :**: L'alliage est ensuite introduit à la spatule dans la soude à la vitesse de 10 g/h, en veillant à ce que la température moyenne du milieu ne dépasse pas 50°C (refroidissement par bain glac-eau).
- **Etape 3 bis :**: Simultanément, on incorpore la solution alcaline contenant le dopant à l'aide d'une pompe doseuse.
- **Etape 4 :**: Lorsque tout l'alliage est introduit, on attend la fin de l'effervescence (5 min).
- **Etape 5 :**: On chauffe à reflux (température = 108°C) pendant 2 heures.
- **Etape 6 :**: Après 2 heures de reflux, on retire le ballon du chauffe-ballon et on attend 5 min que l'ébullition cesse. On place un aimant sous le ballon et on élimine le sumageant après décantation de la phase solide.
- **Etape 7 :**: On introduit 300 ml de soude 1N quasi-bouillante (environ 85°C) et on remue le ballon 3 ou 4 fois. On place alors un aimant sous le ballon et on laisse décanter le solide. Enfin, on élimine le surnageant.
- **Etape 8 :**: On introduit 300 ml de soude 6N quasi-bouillante (environ 85°C) et on porte à reflux pendant 2 heures.
- **Etape 9 :**: idem **étape 6**.
- **Etape 10 :**: idem **étape 7** mais avec 300 ml de soude 6N "bouillante".
- **Etape 11 :**: idem **étape 7** mais avec 300 ml de soude 3N "bouillante".
- **Etape 12 :**: idem **étape 7** mais avec 300 ml de soude 2N "bouillante".
- **Etape 13 :**: idem **étape 7** mais avec 300 ml de soude 1N "bouillante".
- **Etape 14 :**: On récupère le solide dans un flacon et on le stocke dans la soude 1N froide.

La solution alcaline de l'étape 3 bis peut être celle de tartrate de Ti ou de Cr dont les préparations sont décrites en 1.1. et 1.2. ci-dessus.

La quantité de solution alcaline de dopage mise en oeuvre est fonction des concentrations finales visées en dopant.

Ainsi, avec 10 g "d'alliage commercial", on utilise 55,6 ml d'une solution de tartrate de Ti dans la soude 6N à 3,6 g/l en Ti pour obtenir un rapport final Ti/Ni = 1,20 % en poids dans le catalyseur fini.

Divers catalyseurs dopés au Ti et/ou au Cr ont été ainsi préparés à partir de différents alliages, avec différentes concentrations dopant/Ni dans le catalyseur fini.

### EXEMPLES 1 A 25 : HYDROGENATION D'ADIPONITRILE (ADN) EN HEXAMETHYLENEDIAMINE (HMD) A L'AIDE DES CATALYSEURS SELON L'INVENTION ET ESSAIS COMPARATIFS 1 A 4 A L'AIDE DE CATALYSEURS SELON L'ART ANTERIEUR

### 1. APPAREILLAGE, PRODUITS MIS EN OEUVRE ET METHODOLOGIE

### 1.1. Appareillage pour tests discontinus

On utilise un autoclave de 150 ml en acier inoxydable 316 L. Cet autoclave est équipé d'un système d'agitation magnétique (1500 tr/min, barreau magnétique et contrepales) assurant un bon transfert gaz-liquide. Le chauffage se fait au moyen d'un manchon chauffant thermorégulé. Le substrat à hydrogéner est introduit par l'intermédiaire d'une ampoule en acier surmontant l'autoclave ; il peut aussi être introduit à l'aide d'une pompe haute pression dans le cas d'un réacteur semi-continu. L'hydrogène est stocké sous 5 MPa dans une réserve munie d'un manomètre relié à un enregistreur. Il est détendu dans le montage à la pression constante de la réaction. La cinétique de la réaction est suivie par enregistrement de la baisse de pression dans la réserve d'hydrogène. Les échantillons d'hydrogénats destinés à l'analyse sont prélevés par l'intermédiaire d'un tube plongeant muni d'un filtre en acier.

### 1.2. Produits utilisés

- Adiponitrile à 99,99 % (Rhône-Poulenc, PM=108,15).
- Hexaméthylènediamine à 99,99 % (Rhône-Poulenc, PM = 116,21).
- Hydrogène U à 99,995 % en volume.
- Ethanol à 99,8 %.
- Eau distillée
- Soude à 98 % ou Potasse à 86 %.
- Catalyseur : nickel de Raney dopé chimiquement selon l'invention au Ti et/ou au Cr préparé comme décrit précédemment ou dopé métallurgiquement au Cr ou non dopé selon l'art antérieur.

### 1.3. Déroulement d'un test type

### 1.3.1. Charges

Adiponitrile : 6,0 g (0,055 mole).
Hydrogène : excès (0,222 mole).
Milieu réactionnel : 42 g.de solvant réactionnel [HMD/H₂O/éthanol] + base alcaline [NaOH] ; la base alcaline représente 0,10 % en poids du milieu réactionnel ;
42 g de solvant réactionnel [HMD/H₂O] + KOH ; la base alcaline représente 0,05 % en poids du milieu réactionnel.
Catalyseur : 0,40 g.

### 1.3.2. Mode opératoire

On prélève un excès de bouillie de nickel de Raney (1-2g), on lave le catalyseur avec six fois 50 ml d'eau distillée, on pèse exactement 0,40 g de catalyseur au pycnomètre. Le nickel de Raney humide est ensuite introduit dans l'autoclave. Pour une masse de 0,40 g de catalyseur la quantité d'eau couramment entraînée est de l'ordre de 0,4 g. Cette masse d'eau sera prise en compte dans la composition pondérale du solvant réactionnel de 60/30/10 en HMD/éthanol/eau ou 98/2 en HMD/eau. La base alcaline est introduite avec la quantité d'eau nécessaire à l'ajustement des pourcentages en eau requis. L'ensemble de ces manipulations doit se dérouler sous atmosphère d'argon pour minimiser la carbonatation du solvant et l'oxydation du catalyseur.
L'autoclave est ensuite purgé à l'azote et à l'hydrogène. Et il est enfin chauffé et maintenu sous 2,5 MPa d'hydrogène. L'enregistrement de la pression dans la réserve d'hydrogène est mis en route et l'ADN est additionné rapidement. Quand la consommation d'hydrogène devient nulle, le réacteur est encore laissé sous agitation pendant une demi-heure pour mieux assurer la fin de la réaction. En fin, d'essai, un échantillon d'hydrogénat est prélevé pour la détermination de la sélectivité. L'activité initiale et une "activité moyenne" sont déduites de la courbe de consommation d'hydrogène en fonction du temps.

### 2. ANALYSES

### 2.1. Mesure de l'activité

La pente à l'origine de la courbe de consommation d'hydrogène est proportionnelle à la vitesse initiale (Vi). Cette grandeur est calculée en faisant le quotient à l'origine du nombre de moles d'hydrogène consommé par unité de temps ramené à l'unité de masse de catalyseur. La vitesse initiale sera exprimée en kmole d'hydrogène consommé par kg de catalyseur et par seconde.

Pour une bonne appréciation des performances d'un catalyseur, il est nécessaire de savoir si l'activité initiale n'est pas altérée par un vieillissement prématuré. C'est pourquoi on mesure également la vitesse moyenne de réaction (Vm), qui est le quotient du nombre de moles d'hydrogène mises en jeu au temps total de la réaction par unité de masse de catalyseur et par seconde.

La reproductibilité du test pour la détermination de Vi et Vm donne une incertitude inférieure à 10 %.

### 2.2. Mesure de la sélectivité

En fin de réaction un échantillon d'hydrogénat est prélevé et dilué environ 40 fois dans l'isopropanol. Cet échantillon est analysé quantitativement par chromatographie en phase gazeuse (CPG) à l'aide d'une colonne semi-capillaire). Le détecteur est à ionisation de flamme. La détermination quantitative des sous-produits de la réaction d'hydrogénation de l'ADN est réalisée par la méthode de l'étalon interne (undécane).

La liste des principaux sous-produits dosés est donnée ci-après :
HMI : Hexaméthylèneimine
AMCPA : Aminométhylcyclopentylamine
AZCHe : Azacycloheptène
NEtHMD : N - éthylhexaméthylènediamine
DCH : Diaminocyclohexane cis et trans
BHT : Bis(hexaméthylènetriamine).

La sélectivité (S) en HMD en pourcentage est donnée par la relation : 100 - somme des sélectivités des sous-produits. En effet l'HMD étant mise en oeuvre dans le solvant réactionnel, elle ne peut pas être dosée directement de manière très précise. Par contre il a été vérifié que les sous-produits sont globalement tous identifiés.

Les sélectivités en chacun des sous-produits sont représentées par le pourcentage molaire du sous-produit formé par rapport à l'ADN transformé. Dans tous les exemples et essais comparatifs effectués, le taux de transformation de l'ADN (ainsi que celui de l'aminocapronitrile intermédiaire) est de 100 %.

Lorsqu'un composé n'atteint pas sa limite de détection, la mention ND (non détecté) sera portée dans les tableaux de résultats.

Le taux d'insaturés présent dans l'hydrogénat peut être évalué par polarographie.

### 3. RESULTATS

### 3.1. Catalyseurs Ni de Raney dopés au titane

Hydrogénation avec HMD/H₂O/éthanol/NaOH (exemples 1à 16).

Le tableau 1 ci-après rassemble les résultats obtenus dans ces exemples ainsi que dans un essai comparatif (Ec) 1 mettant en oeuvre un catalyseur Ni de Raney non dopé et les essais comparatifs (Ec) 2 et 3 mettant en oeuvre des Ni de Raney dopés métallurgiquement au chrome.

### 3.2. Catalyseurs Ni de RANEY doués au chrome

Hydrogénation avec HMD/H₂O/éthanol/NaOH (exemples 17 à 20 et essai comparatif 4 avec Ni de Raney dopé métallurgiquement au Cr).

### 3.3. Catalyseurs Ni de Raney doués au titane et au chrome

Hydrogénation avec HMD/H₂O/éthanol/NaOH (exemples 21 à 25).

Le tableau 2 ci-après rassemble les résultats obtenus dans les exemples 17 à 25 et fait apparaître d'une part que le chrome est un dopant tout aussi avantageux que le titane et d'autre part que la mise en oeuvre de deux éléments dopants est, elle aussi, tout à fait avantageuse, au regard de la sélectivité et de l'élimination des impuretés. On note également que les catalyseurs dopés métallurgiquement conduisent à des sélectivités en sous-produits, tels que BHT, HMI notamment, plus élevées que les catalyseurs dopés chimiquement selon l'invention.

### 3.4. Catalyseur Ni de Raney doué au titane

Hydrogénation avec HMD/H₂O/KOH (exemple 26).
- alliage de départ : Ni/Al = 50/50 en poids
- 0,72 % en poids de Ti/Ni dans le catalyseur
- 3,25 % en poids de Al/Ni dans le catalyseur.

Les résultats suivants ont été obtenus :
- Vi = 46
- Vm = 4
- S en HMD : 98,0%
- S en HMI : 0,203 %
- S en AzCHE : 0,191 %
- S en DCH : 0,046 %
- S en AMCPA : 0,489 %
- S en BHT : 1,130 %.

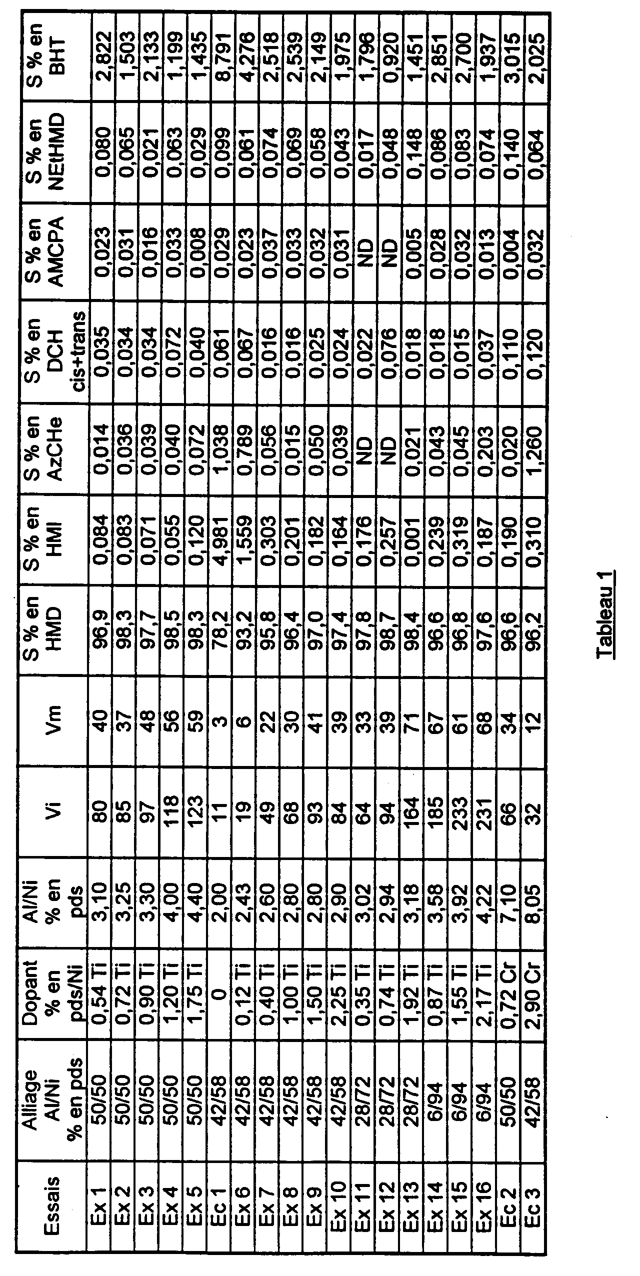

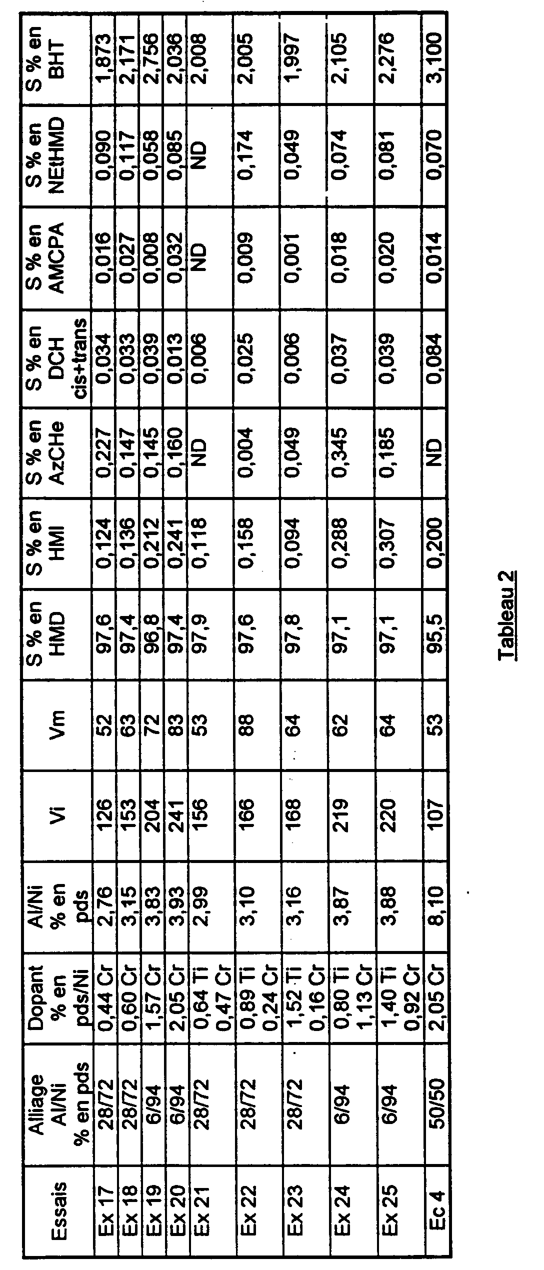

## Revendications

1. Procédé de préparation d'un catalyseur du type Ni de Raney utilisable pour l'hydrogénation de nitriles en amines, ledit catalyseur étant dopé avec au moins un élément choisi parmi les éléments des groupes IIB, IVB à VIIB de la classification périodique, procédé dans lequel on soumet un alliage métallique comprenant du nickel et de l'aluminium à une attaque alcaline, caractérisé en ce qu'il consiste :
- à mettre en oeuvre un alliage métallique Ni/Al sensiblement exempt dopant
- et à effectuer l'attaque alcaline de l'alliage en présence d'une solution comprenant le dopant sous forme complexée avec au moins un chélatant.

2. Procédé selon la revendication 1, caractérisé en ce que l'on introduit le dopant, dans le milieu d'attaque alcaline, en solution de préférence alcaline et plus préférentiellement encore de même nature et sensiblement de même titre alcalin que le milieu d'attaque.

3. Procédé selon l'une des revendications 1 ou 2, caractérisé en ce que l'élément dopant est sous forme de complexe avec au moins un chélatant, sélectionné parmi les dérivés d'acides carboxyliques, les triènes, les amines ou autres séquestrants appropriés.

4. Procédé selon l'une des revendications 1 à 3, caractérisé en ce que l'élément dopant est sous forme de complexe avec au moins un chélatant sélectionné parmi les composés suivants : tartrate, citrate, éthylènediaminetétraacétate, gluconate, carboxylates d'acides gras tel que stéarate, et est de préférence un tartrate.

5. Procédé selon l'une des revendications 1 à 4, caractérisé en ce que le ou les éléments dopants sont choisis parmi les éléments suivants : titane, chrome, zirconium, vanadium, molybdène, manganèse, zinc et de préférence parmi le titane, le chrome et le zirconium.

6. Procédé selon l'une des revendications 1 à 5, caractérisé en ce que l'on introduit le dopant dès le début de l'attaque alcaline.

7. Procédé selon l'une des revendications 1 à 6, caractérisé en ce que l'on fait intervenir plusieurs dopants lors de l'attaque alcaline de l'alliage précurseur.

8. Catalyseur susceptible d'être obtenu par le procédé selon l'une des revendications 1 à 7, caractérisé en ce qu'il comprend une teneur en aluminium, exprimée en poids par rapport au poids du nickel, inférieure ou égale à 6 %, de préférence inférieure ou égale à 5 % et, plus préférentiellement encore, comprise entre 2,5 % et 4,5 %.

9. Catalyseur selon la revendication 8, caractérisé en ce que le rapport pondéral dopant/Ni est compris entre 0,05 % et 10 %, de préférence entre 0,1 % et 5 %, et, plus préférentiellement encore, entre 0,3 % et 3,5 %.

10. Procédé d'hydrogénation de nitriles en amines, caractérisé en ce que l'on met en oeuvre un catalyseur selon l'une des revendications 8 ou 9.

11. Procédé selon la revendication 10, caractérisé en ce que le substrat nitrile mis en oeuvre est un dinitrile de formule (I) :
NC―R―CN (I)
dans laquelle R représente un groupement alkylène ou alcénylène, linéaire ou ramifié, ayant de 1 à 12 atomes de carbone, ou un groupement arylène ou aralkylène ou aralcénylène substitué ou non,
et de préférence un nitrile de formule (I) dans laquelle R représente un radical alkylène, linéaire ou ramifié ayant de de 2 à 6 atomes de carbone.

12. Procédé selon l'une des revendications 10 ou 11, caractérisé en ce que le substrat nitrile est choisi parmi l'adiponitrile, le méthylglutaronitrile, l'éthylsuccinonitrile, le malononitrile, le succinonitrile et le glutaronitrile et leurs mélanges.

13. Procédé selon l'une quelconque des revendications 10 à 12, caractérisé en ce que le substrat, caractérisé en ce que l'on fixe la concentration en substrat nitrile dans le milieu réactionnel total à une valeur comprise entre 0,001 % et 30 % en poids par poids et de préférence entre 0,1 % et 20 %.

14. Procédé selon l'une quelconque des revendications 10 à 13, caractérisé en ce que la base mise en oeuvre est constituée par au moins l'un des composés suivants : LiOH, NaOH, KOH, RbOH, CsOH.

15. Procédé selon l'une quelconque des revendications 10 à 14, caractérisé en ce que le milieu réactionnel liquide comprend de l'eau, de préférence dans une quantité inférieure ou égale à 20 % en poids du milieu réactionnel liquide total et, plus préférentiellement encore, comprise entre 0,1 % et 15 % en poids.

16. Procédé selon l'une quelconque des revendications 10 à 15, caractérisé en ce que le milieu réactionnel liquide contient de l'amine visée.

17. Procédé selon la revendication 16, caractérisé en ce que l'amine visée est introduite dans le milieu réactionnel liquide, à raison de 50 à 99 % et préférentiellement à raison de 60 à 99 % en poids par rapport au poids du milieu réactionnel liquide total.

18. Procédé selon l'une des revendications 10 à 17, caractérisé en ce que l'on met en oeuvre un milieu réactionnel liquide comprenant un alcool comme le méthanol, l'éthanol, le propanol, l'isopropanol, le butanol, les glycols tels que éthylèneglycol et/ou propylène glycol, les polyols et leurs mélanges et/ou un amide tel que le diméthylformamide et/ou le diméthylacétamide.

19. Procédé selon l'une quelconque des revendications 10 à 17, caractérisé en ce que l'on utilise la base dans une quantité supérieure ou égale à 0,1 mol/kg de catalyseur, de préférence comprise entre 0,1 et 2,0 mol/kg de catalyseur et, plus préférentiellement encore, entre 0,5 et 1,5 mol/kg de catalyseur.

20. Procédé selon la revendication 18, caractérisé en ce que l'on utilise la base dans une quantité supérieure ou égale à 0,05 mol/kg de catalyseur, de préférence comprise entre 0,1 et 10,0 mol/kg et, plus préférentiellement encore, entre 1,0 et 8,0 mol/kg.

21. Procédé selon l'une quelconque des revendications 10 à 20, caractérisé en ce que l'on effectue l'hydrogénation à une température de milieu réactionnel inférieure ou égale à 150°C, de préférence inférieure ou égale à 120°C et, plus préférentiellement encore, inférieure ou égale à 100°C.

22. Procédé selon l'une des revendications 10 à 21, caractérisé en ce que le dinitrile mis en oeuvre est l'adiponitrile et en ce que ce dernier est transformé en hexaméthylènediamine.

23. Procédé selon l'une des revendications 10 à 21, caractérisé en ce que le dinitrile mis en oeuvre est l'adiponitrile et en ce que ce dernier est transformé en aminocapronitrile.

## Claims

1. Process for the preparation of a catalyst of Raney Ni type which can be used for the hydrogenation of nitriles to amines, the said catalyst being doped with at least one element chosen from the elements of groups IIB and IVB to VIIB of the periodic classification, in which process a metal alloy comprising nickel and aluminium is subjected to an alkaline attack, characterized in that it consists:
- in using an Ni/Al metal alloy which is substantially free from doping agent,
- and in carrying out the alkaline attack on the alloy in the presence of a solution comprising the doping agent in the form complexed with at least one chelating agent.

2. Process according to Claim 1, characterized in that the doping agent is introduced into the alkaline attack medium as a solution which is preferably alkaline and more preferentially still of the same nature and substantially of the same alkaline assay as the attack medium.

3. Process according to either of Claims 1 and 2, characterized in that the doping element is in the form of a complex with at least one chelating agent selected from carboxylic acid derivatives, trienes, amines or other appropriate sequestering agents.

4. Process according to one of Claims 1 to 3, characterized in that the doping element is in the form of a complex with at least one chelating agent selected from the following compounds: tartrate, citrate, ethylenediaminetetraacetate, gluconate or fatty acid carboxylates such as stearate, and is preferably a tartrate.

5. Process according to one of Claims 1 to 4, characterized in that the doping element(s) is/are chosen from the following elements: titanium, chromium, zirconium, vanadium, molybdenum, manganese or zinc and preferably from titanium, chromium and zirconium.

6. Process according to one of Claims 1 to 5, characterized in that the doping agent is introduced from the beginning of the alkaline attack.

7. Process according to one of Claims 1 to 6, characterized in that a number of doping agents are involved during the alkaline attack on the precursor alloy.

8. Catalyst capable of being obtained by the process according to one of Claims 1 to 7, characterized in that it comprises an aluminium content, expressed by weight with respect to the weight of the nickel, of less than or equal to 6%, preferably of less than or equal to 5% and, more preferentially still, of between 2.5% and 4.5%.

9. Catalyst according to Claim 8, characterized in that the doping agent/Ni ratio by weight is between 0.05% and 10%, preferably between 0.1% and 5% and, more preferentially still, between 0.3% and 3.5%.

10. Process for the hydrogenation of nitriles to amines, characterized in that use is made of a catalyst according to either of Claims 8 and 9.

11. Process according to Claim 10, characterized in that the nitrile substrate used is a dinitrile of formula (I) :
NC-R-CN (I)
in which R represents a linear or branched alkylene or alkenylene group having from 1 to 12 carbon atoms or a substituted or unsubstituted arylene or aralkylene or aralkenylene group,
and preferably a nitrile of formula (I) in which R represents a linear or branched alkylene radical having from from 2 to 6 carbon atoms.

12. Process according to either of Claims 10 and 11, characterized in that the nitrile substrate is chosen from adiponitrile, methylglutaronitrile, ethylsuccinonitrile, malononitrile, succinonitrile and glutaronitrile and their mixtures.

13. Process according to any one of Claims 10 to 12, characterized in that the concentration of nitrile substrate in the total reaction medium is set at a value of between 0.001% and 30% weight for weight and preferably between 0.1% and 20%.

14. Process according to any one of Claims 10 to 13, characterized in that use is made of a base consisting of at least one of the following compounds: LiOH, NaOH, KOH, RbOH or CsOH.

15. Process according to any one of Claims 10 to 14, characterized in that the liquid reaction medium comprises water, preferably in an amount less than or equal to 20% by weight of the total liquid reaction medium and, more preferentially still, between 0.1% and 15% by weight.

16. Process according to any one of Claims 10 to 15, characterized in that the liquid reaction medium contains targeted amine.

17. Process according to Claim 16, characterized in that the targeted amine is introduced into the liquid reaction medium in a proportion of 50 to 99% and preferentially in a proportion of 60 to 99% by weight with respect to the weight of the total liquid reaction medium.

18. Process according to any one of Claims 10 to 17, characterized in that use is made of a liquid reaction medium comprising an alcohol such as methanol, ethanol, propanol, isopropanol, butanol, glycols, such as ethylene glycol and/or propylene glycol, polyols and their mixtures and/or an amide such as dimethylformamide and/or dimethylacetamide.

19. Process according to any one of Claims 10 to 17, characterized in that the base is used in an amount greater than or equal to 0.1 mol/kg of catalyst, preferably between 0.1 and 2.0 mol/kg of catalyst and, more preferentially still, between 0.5 and 1.5 mol/kg of catalyst.

20. Process according to Claim 18, characterized in that the base is used in an amount greater than or equal to 0.05 mol/kg of catalyst, preferably between 0.1 and 10.0 mol/kg and, more preferentially still, between 1.0 and 8.0 mol/kg.

21. Process according to any one of Claims 10 to 20, characterized in that the hydrogenation is carried out at a temperature of the reaction medium which is less than or equal to 150°C, preferably less than or equal to 120°C and, more preferentially still, less than or equal to 100°C.

22. Process according to one of Claims 10 to 21, characterized in that the dinitrile used is adiponitrile and in that the latter is converted to hexamethylenediamine.

23. Process according to one of Claims 10 to 21, characterized in that the dinitrile used is adiponitrile and in that the latter is converted to aminocapronitrile.

## Patentansprüche

1. Verfahren zur Herstellung eines Katalysators vom Typ Raney-Nickel, verwendbar für die Hydrierung von Nitrilen zu Aminen, wobei der genannte Katalysator mit mindestens einem Element dotiert ist, ausgewählt unter den Elementen der Gruppen IIB, IVB bis VIIB des Periodensystems der Elemente, wobei man bei dem Verfahren eine Nickel und Aluminium umfassende metallische Legierung einem alkalischen Angriff unterzieht, dadurch gekennzeichnet, daß es darin besteht:
- eine nahezu von dem Dotierungs-Element freie metallische Legierung Ni/Al einzusetzen, und
- den alkalischen Angriff auf die Legierung in Anwesenheit einer Lösung durchzuführen, die das Dotierungs-Element in Form eines Komplexes mit mindestens einem Chelatbildner umfaßt.

2. Verfahren nach Anspruch 1, dadurch gekennzeichnet, daß man das Dotierungs-Element in das Milieu des alkalischen Angriffs einträgt, in vorzugsweise alkalischer Lösung und noch bevorzugter von der gleichen Beschaffenheit und mit etwa dem gleichen alkalischen Titer wie das Angriffsmilieu.

3. Verfahren nach einem der Ansprüche 1 oder 2, dadurch gekennzeichnet, daß das Dotierungs-Element in Form eines Komplexes mit mindestens einem Chelatbildner vorliegt, ausgewählt unter den Derivaten von Carbonsäuren, den Trienen, den Aminen oder anderen geeigneten Maskierungsmitteln.

4. Verfahren nach einem der Ansprüche 1 bis 3, dadurch gekennzeichnet, daß das Dotierungs-Element in Form eines Komplexes mit mindestens einem Chelatbildner vorliegt, ausgewählt unter den folgenden Verbindungen: Tartraten, Citraten, Ethylendiamintetraacetaten, Gluconaten, Carboxylaten von Fettsäuren wie Stearaten, und der Chelatbildner vorzugsweise ein Tartrat ist.

5. Verfahren nach einem der Ansprüche 1 bis 4, dadurch gekennzeichnet, daß das oder die Dotierungs-Element(e) unter den folgenden Elementen ausgewählt werden: Titan, Chrom, Zirkonium, Vanadium, Molybdän, Mangan, Zink und vorzugsweise unter Titan, Chrom und Zirkonium.

6. Verfahren nach einem der Ansprüche 1 bis 5, dadurch gekennzeichnet, daß man das Dotierungs-Element schon ab dem Beginn des alkalischen Angriffs an einträgt.

7. Verfahren nach einem der Ansprüche 1 bis 6, dadurch gekennzeichnet, daß man bei dem alkalischen Angriff auf die Vorläufer-Legierung mehrere Dotierungs-Elemente einsetzt.

8. Katalysator, geeignet gemäß dem Verfahren nach einem der Ansprüche 1 bis 7 erhalten zu werden, dadurch gekennzeichnet, daß er einen Gehalt an Aluminium, ausgedrückt in Gewicht bezogen auf das Gewicht von Nickel, von unterhalb oder gleich 6 %, vorzugsweise von unterhalb oder gleich 5 % und noch bevorzugter zwischen 2,5 % und 4,5 % umfaßt.

9. Katalysator nach Anspruch 8, dadurch gekennzeichnet, daß das Gewichtsverhältnis Dotierungs-Element/Ni zwischen 0,05 % und 10 %, vorzugsweise zwischen 0,1 % und 5 % und noch bevorzugter zwischen 0,3 % und 3,5 % liegt.

10. Verfahren zur Hydrierung von Nitrilen zu Aminen, dadurch gekennzeichnet, daß man einen Katalysator nach einem der Ansprüche 8 oder 9 einsetzt.

11. Verfahren nach Anspruch 10, dadurch gekennzeichnet, daß das eingesetzte Nitrilsubstrat ein Dinitril der Formel (I)
NC-R-CN (I)
ist, in der R eine gerade oder verzweigte Gruppe Alkylen oder Alkenylen mit 1 bis 12 Kohlenstoffatomen oder eine substituierte oder nicht substituierte Gruppe Arylen oder Aralkylen oder Aralkenylen darstellt, und vorzugsweise ein Nitril der Formel (I) ist, in der R einen geraden oder verzweigten Rest Alkylen mit 2 bis 6 Kohlenstoffatomen bedeutet.

12. Verfahren nach einem der Ansprüche 10 oder 11, dadurch gekennzeichnet, daß das Nitrilsubstrat unter Adiponitril, Methylglutaronitril, Ethylsuccinonitril, Malononitril, Succinonitril und Glutaronitril sowie deren Mischungen ausgewählt wird.

13. Verfahren nach irgendeinem der Ansprüche 10 bis 12, dadurch gekennzeichnet, daß das Substrat dadurch gekennzeichnet ist, daß man die Konzentration an Nitrilsubstrat in dem gesamten Reaktionsmilieu auf einen Wert zwischen 0,001 Gew.-% und 30 Gew.-% pro Gewicht und vorzugsweise zwischen 0,1 % und 20 % festlegt.

14. Verfahren nach irgendeinem der Ansprüche 10 bis 13, dadurch gekennzeichnet, daß die eingetzte Base aus mindestens einer der folgenden Verbindungen besteht: LiOH, NaOH, KOH, RbOH, CsOH.

15. Verfahren nach irgendeinem der Ansprüche 10 bis 14, dadurch gekennzeichnet, daß das flüssige Reaktionsmilieu Wasser umfaßt, vorzugsweise in einer Menge von unterhalb oder gleich 20 Gew.-% des gesamten flüssigen Reaktionsmilieus und noch bevorzugter zwischen 0,1 Gew.-% und 15 Gew.-%.

16. Verfahren nach irgendeinem der Ansprüche 10 bis 15, dadurch gekennzeichnet, daß das flüssige Reaktionsmilieu das angestrebte Amin enthält.

17. Verfahren nach Anspruch 16, dadurch gekennzeichnet, daß das angestrebte Amin in das flüssige Reaktionsmilieu in einem Verhältnis von 50 Gew.-% bis 99 Gew.-% und vorzugsweise in einem Verhältnis von 60 Gew.-% bis 99 Gew.-%, bezogen auf das Gewicht des gesamten flüssigen Reaktionsmilieu, eingetragen wird.

18. Verfahren nach einem der Ansprüche 10 bis 17, dadurch gekennzeichnet, daß man ein flüssiges Reaktionsmilieu einsetzt, das einen Alkohol wie Methanol, Ethanol, Propanol, Isopropanol, Butanol, Glycole wie Ethylenglycol und/oder Propylenglycol, Polyole und ihre Mischungen und/oder ein Amid wie Dimethylformamid und/oder Dimethylacetamid umfaßt.

19. Verfahren nach irgendeinem der Ansprüche 10 bis 17, dadurch gekennzeichnet, daß man die Base in einer Menge von höher oder gleich 0,1 Mol/kg Katalysator, vorzugsweise zwischen 0,1 Mol/kg und 2,0 Mol/kg Katalysator und noch bevorzugter zwischen 0,5 Mol/kg und 1,5 Mol/kg Katalysator verwendet.

20. Verfahren nach Anspruch 18, dadurch gekennzeichnet, daß man die Base in einer Menge von höher oder gleich 0,05 Mol/kg Katalysator, vorzugsweise zwischen 0,1 Mol/kg und 10,0 Mol/kg und noch bevorzugter zwischen 1,0 Mol/kg und 8,0 Mol/kg Katalysator verwendet.

21. Verfahren nach irgendeinem der Ansprüche 10 bis 20, dadurch gekennzeichnet, daß man die Hydrierung bei einer Temperatur des Reaktionsmilieus von unterhalb oder gleich 150 °C, vorzugsweise von unterhalb oder gleich 120 °C und noch bevorzugter von unterhalb oder gleich 100 °C durchführt.

22. Verfahren nach einem der Ansprüche 10 bis 21, dadurch gekennzeichnet, daß das eingesetzte Dinitril Adiponitril ist und dadurch, daß dieses letztere in Hexamethylendiamin umgewandelt wird.

23. Verfahren nach einem der Ansprüche 10 bis 21, dadurch gekennzeichnet, daß das eingesetzte Dinitril Adiponitril ist und dadurch, daß dieses letztere in Aminocapronitril umgewandelt wird.
